# EUROPÄISCHE PATENTSCHRIFT

(11) **EP 0 916 323 B1**
(45) Veröffentlichungstag und Bekanntmachung des Hinweises auf die Patenterteilung: **02.01.2003**
(21) Anmeldenummer: 98121070.1
(22) Anmeldetag: 06.11.1998
(51) Int. Cl.: A61F 2/44

(54) **Zwischenwirbelimplantat**
Intervertebral implant
Implant intervertébral

(30) Priorität: 12.11.1997 DE 29720022 U
(43) Veröffentlichungstag der Anmeldung: 19.05.1999
(73) Patentinhaber: Schäfer, Bernd, 6315 Oberägeri (CH)
(72) Erfinder: Schäfer, Bernd, 73035 Göppingen (DE); Halm, Henry Dr., 49143 Bissendorf-Wissingen (DE)
(74) Vertreter: Dreiss, Fuhlendorf, Steimle & Becker

(56) Entgegenhaltungen:
- EP-A- 0 599 419
- WO-A-96/40014
- WO-A-97/32547
- DE-U- 29 720 022
- FR-A- 2 736 537
- US-A- 4 820 305

## Beschreibung

Die Erfindung betrifft ein Zwischenwirbelimplantat zum Einsatz zwischen zwei Wirbelkörper einer Wirbelsäule mit den Merkmalen des Oberbegriffs des Anspruchs 1.

Die einzelnen Wirbel der Wirbelsäule weisen u.a. einen Wirbelkörper, einen Wirbelbogen, einen Dornfortsatz, zwei Querfortsätze und zwei obere und zwei untere Gelenkfortsätze auf. Die Wirbel sind über an ihren Wirbelkörpern (corpus vertebrae) anliegenden Zwischenwirbelscheiben (disci intervertebralis) miteinander verbunden. Diese Zwischenwirbelscheiben bestehen aus flüssigkeitsreichem Faserknorpel und verbinden die einzelnen Wirbelkörper miteinander. Die Größe der Zwischenwirbelscheiben nimmt von oben nach unten entsprechend der im menschlichen Körper auftretenden Belastung zu. Die Zwischenwirbelscheiben dienen als elastische Puffer und dämpfen federnd Stöße ab. Es ist bekannt, daß sich die Zwischenwirbelscheiben verlagern können oder daß der innere Gallertkern (nucleus pulposus) durch Risse im bindegewebeartigen knorpeligen äußeren Ring (annulus fibrosus) austreten kann. Dabei kann die Zwischenwirbelscheibe teilweise in die Zwischenwirbellöcher (foramina intervertebralia) bzw. in den Spinalkanal eintreten. Außerdem kann dieser Prolaps medial bzw. dorsal medial oder lateral sein. Derartige Prolapse treten am häufigsten an den L₄-L₅, L₅-S₁ und C₆-C₇-Vertebrales auf. Werden derartige Prolapse nicht therapiert, kommt es zu irreversiblen Druckschädigungen von Nervenwurzeln oder zu Querschnittsläsionen. Sollte eine symptomatische Physiotherapie, z.B. Krankengymnastik oder Massage, keinen Erfolg versprechen, muss die discus intervertebralis operativ entfernt werden. Nun besteht die Möglichkeit der Implantation einer künstlichen Zwischenwirbelscheibe oder der Osteosynthese der beiden Wirbel über ein starres Zwischenwirbelimplantat.

Aus der EP-A-0 392 076 ist eine künstliche Zwischenwirbelscheibe bekannt geworden, die aus einer oberen und unteren Anlagefläche und einer elastischen Zwischenschicht besteht. Aus der Anlagefläche ragen Ankerbolzen heraus, über die die künstliche Zwischenwirbelscheibe an den Wirbelkörpern fixiert wird. Als nachteilig hat sich herausgestellt, dass aufgrund der elastischen Zwischenschicht zwischen den beiden Anlageflächen keine optimale Synthese der beiden Wirbel erzielbar ist.

Aus der US-A- 5,192,327 ist ein starres Zwischenwirbelimplantat bekannt geworden, welches zur Osteosynthese ebenfalls zwischen zwei Wirbelkörper eingesetzt wird. Bei diesem Implantat müssen die beiden Wirbel auf einen vorgegebenen Abstand zueinander gebracht werden, um das Implantat einsetzen zu können. Weisen die beiden Wirbel einen zu großen Abstand auf, kann das Implantat leicht verrutschen bzw. findet keinen Halt.

Beiden Zwischenwirbelimplantaten haftet jedoch der Nachteil an, dass die Operation zum Einsetzen dieser Zwischenwirbelimplantate ventral erfolgen muss. Dies ist nicht nur zeit- und kostenaufwendig sondern belastet auch stark den Patienten, da durchaus die Möglichkeit besteht, dass die Wirbelsäule posterior stabilisiert wird und dennoch ein ventraler Eingriff zum Einsetzen des Zwischenwirbelimplantats erforderlich ist.

Aus der WO-A-96 40 014 ist ein Zwischenwirbelimplantat bekannt geworden, welches dorsal verwendbar ist. Jedoch ist die Handhabung, insbesondere das Befestigen eines Werkzeugs schwierig.

Aus der FR-A-2 736 537 ist ein Implantat bekannt geworden, welches an einer Querseitenwand eine Gewindebohrung aufweist. Das Implantat ist aber asymmetrisch geformt und daher nicht universell verwendbar.

Der Erfindung liegt daher die Aufgabe zugrunde, ein Zwischenwirbelimplantat bereitzustellen, welches universell verwendbar ist und das mittels des Werkzeugs korrekt und problemlos einsetzbar ist.

Diese Aufgabe wird erfindungsgemäß mit einem Zwischenwirbelimplantat gelöst, das die Merkmale des Anspruchs 1 aufweist.

Mit einem derart ausgebildeten, erfindungsgemäßen Zwischenwirbelimplantat wird der wesentliche Vorteil . geschaffen, dass bei einem dorsalen Eingriff dieses Zwischenwirbelimplantat dorsal in den Körper eingeführt und ventral zwischen die beiden Wirbelkörper eingesetzt werden kann. Zuvor werden die beiden Wirbelkörper mittels Knochenschrauben und entsprechenden Fixiereinrichtungen von dorsaler Seite stabilisiert und ein genügend großer Abstand zwischen den beiden Wirbelkörpern geschaffen, so dass das Zwischenwirbelimplantat eingeführt werden kann. Nimmt dieses seine korrekte Lage ein, dann werden die beiden Wirbelkörper komprimiert und fixiert und dadurch das Zwischenwirbelimplantat gehalten. Dieser Vorgang kann ausschließlich durch einen posterioren chirurgischen Eingriff erfolgen, was den Patienten wesentlich weniger belastet und außerdem mit einem geringeren Risiko verbunden ist, als ein Eingriff von dorsaler sowie von ventraler Seite.

Dabei ist die Wandstärke beider Querseitenwände größer als die Wandstärke der Längsseitenwände. Dabei ist die, die größere Wandstärke aufweisende Querseitenwand mit einer Instrumentenaufnahme versehen, wobei die Instrumentenaufnahme von einer ein Innengewinde aufweisenden Bohrung gebildet wird. In diese Bohrung kann ein Instrument eingeschraubt werden, mit dem das Zwischenwirbelimplantat von dorsal in den Körper eingeführt und von ventraler Seite zwischen die beiden Wirbelkörper eingesetzt werden kann. Sobald das Zwischenwirbelimplantat seine korrekte Lage einnimmt, wird das Instrument von der Instrumentenaufnahme gelöst und aus dem Körper entfernt. Das Zwischenwirbelimplantat kann nun fixiert werden.

Vorteilhaft befindet sich die Instrumentenaufnahme an einem Übergangsbereich von einer Längsseitenwand zu einer Querseitenwand. Dieser Übergangsbereich von der Querseitenwand zur Längsseitenwand hat den wesentlichen Vorteil, dass das vom Zwischenwirbelimplantat abragende Instrument ein korrektes Einsetzen des Zwischenwirbelimplantats zwischen die beiden Wirbelkörper in die gewünschte Position erlaubt, ohne dass das Instrument mit Teilen der Wirbelsäule kollidiert.

Bei einer bevorzugten Ausführungsform ist die Seitenwand des Hohlkörpers bohnenförmig gekrümmt, wobei die beiden Längsseitenwände den gleichen Krümmungsradius aufweisen. Es sind jedoch auch Zwischenwirbelimplantate denkbar, bei denen die konvex gekrümmte Längsseitenwand einen größeren Krümmungsradius aufweist, als die konkav gekrümmte Längsseitenwand.

Vorteilhaft ist die Seitenwand mit Durchbrüchen versehen. Dieser Aufbau hat den wesentlichen Vorteil, dass er relativ einfach ist, ein geringes Gewicht aufweist und von Knochengewebe leicht durchwachsen werden kann. Die Durchbrüche werden vorteilhaft von Bohrungen gebildet.
Dabei weist die Seitenwand zwei Reihen von Durchbrüchen auf, wobei die beiden Reihen von Durchbrüchen symmetrisch zueinander liegen. Auf diese Weise wird trotz des geringen Materialbedarfs dennoch ein stabiler Aufbau gewährleistet, so dass auch hohe Kräfte abgestützt werden können.

Vorteilhaft sind die Querseitenwände und/oder Längsseitenwände kreisbogenförmig gekrümmt, so dass das Zwischenwirbelimplantat sich optimal an die Form der Wirbelkörper anpasst.

Dabei befindet sich der die Instrumentenaufnahme aufweisende Übergangsbereich erfindungsgemäß am Übergang von der konkav gekrümmten Längsseitenwand zur konvex gekrümmten Querseitenwand, wobei die Instrumentenaufnahme in der Querseitenwand liegt.

Das Zwischenwirbelimplantat ist keilförmig ausgebildet, so dass die beiden Anlageflächen in winklig zueinander stehenden Ebenen liegen. Auf diese Weise wird die Stellung der beiden miteinander zu fixierenden Wirbelkörper im wesentlichen beibehalten. Bevorzugt ist die Höhe der konkav gekrümmten Längsseitenwand geringer als die Höhe der konvex gekrümmten Seitenwand. Der Unterschied dieser beiden Wandhöhen kann z.B. 1 mm betragen.

Beim erfindungsgemäßen Zwischenwirbelimplantat kommt vorteilhaft die konkav gekrümmte Längsseitenwand dorsal zu liegen. Um das Zwischenwirbelimplantat sterilisieren zu können wird dieses aus Metall und/oder Kunststoff und/oder Keramik hergestellt.

Weitere Vorteile, Merkmale und Einzelheiten der Erfindung ergeben sich aus den Unteransprüchen sowie aus der nachfolgenden Beschreibung, in der unter Bezugnahme auf die Zeichnung ein bevorzugtes Ausführungsbeispiel beschrieben ist. Dabei können die in den Ansprüchen und in der Beschreibung erwähnten sowie die in Zeichnung dargestellten Merkmale jeweils einzeln für sich oder in beliebiger Kombination erfindungswesentlich sein. In der Zeichnung zeigen:
- Figur 1: eine perspektivische Ansicht einer bevorzugten Ausführungsform des erfindungsgemäßen Zwischenwirbelimplantats;
- Figur 2: eine Seitenansicht des Zwischenwirbelimplantats gemäß Figur 1;
- Figur 3: eine Draufsicht auf das Zwischenwirbelimplantat;
- Figur 4: eine vergrößerte Darstellung IV der linken Querseitenwand des Zwischenwirbelimplantats gemäß Figur 3; und
- Figur 5: eine Seitenansicht des Zwischenwirbelimplantats auf die Querseitenwand.

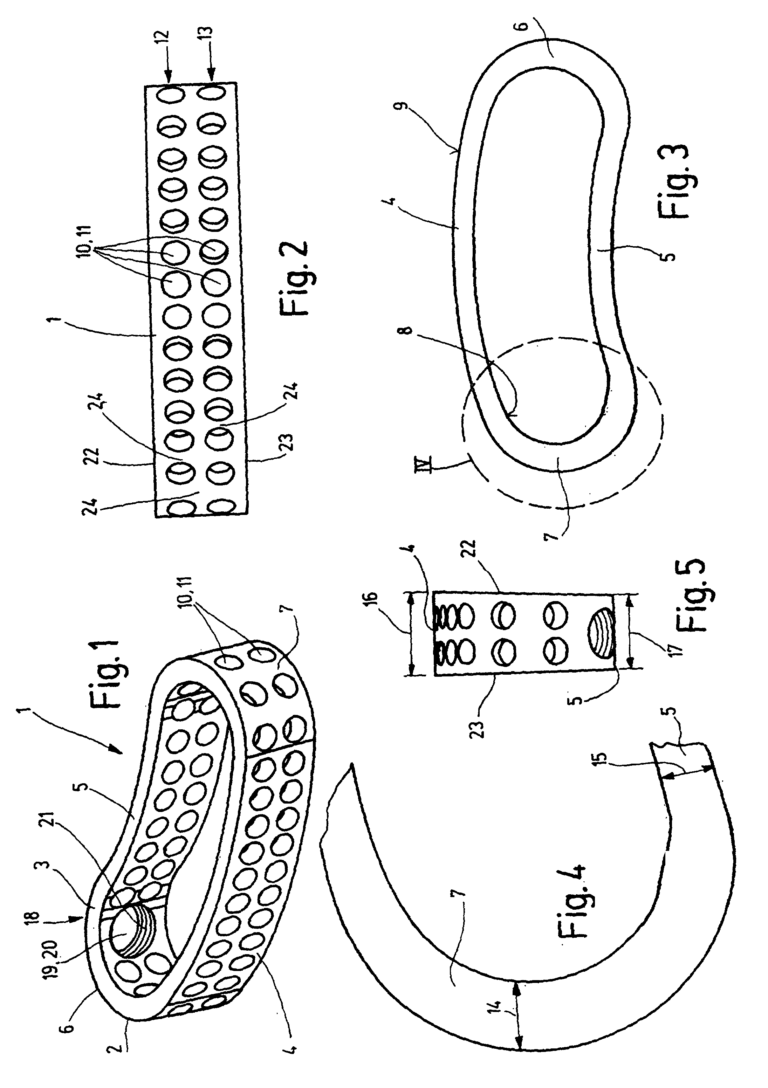

In der Figur 1 ist ein Ausführungsbeispiel des insgesamt mit 1 bezeichneten erfindungsgemäßen Zwischenwirbelimplantats in perspektivischer Ansicht dargestellt. Dieses Zwischenwirbelimplantat 1 ist als Hohlkörper 2 ausgebildet, wobei der Hohlkörper 2 von einer Seitenwand 3 gebildet wird. Diese Seitenwand 3 besteht aus zwei Längsseitenwänden 4 und 5 sowie zwei Querseitenwänden 6 und 7. Die Längsseitenwände 4 und 5 und die Querseitenwände 6 und 7 gehen derart ineinander über, so dass sie eine geschlossene Form, nämlich die Seitenwand 3 bilden. Die Längsseitenwand 4 ist konvex und die Längsseitenwand 5 konkav gekrümmt. Außerdem sind die Querseitenwände 6 und 7 konvex gekrümmt.

Wie sich sehr deutlich aus Figur 3 ergibt, sind die Krümmungsradien der Querseitenwände 6 und 7 kleiner als die Krümmungsradien der Längsseitenwände 4 und 5. Der Krümmungsradius der Innenseite 8 der Querseitenwände beträgt z.B. 4,6 mm. Außerdem ist der Krümmungsradius der Außenseite 9 der Längsseitenwand 4 mit etwa 50 mm größer als der Krümmungsradius der Außenseite 9 der Längsseitenwand 5 mit etwa 35 mm. Aus den Figuren 1 und 3 ist außerdem deutlich erkennbar, dass die Form des erfindungsgemäßen Zwischenwirbelimplantats 1 in etwa bohnenförmig ist.

Die Seitenwand 3 ist mit Durchbrechungen 10 bildenden Bohrungen 11 versehen, welche in zwei Reihen 12 und 13 (Figur 2) angeordnet sind. Außerdem ist die Anordnung der Bohrungen 11 in den Reihen 12 und 13 symmetrisch zueinander, d.h. über jeder unteren Bohrung 11 liegt eine darüberliegende weitere Bohrung 11, erkennbar. Die beiden Anlageflächen 22 und 23 stützen sich also direkt über Stege 24 gegeneinander ab.

Aus Figur 4 ergibt sich deutlich, dass die Wandstärke 14 der Querseitenwand 7 größer ist als die Wandstärke 15 der Längsseitenwand 5. Außerdem ist die Wandstärke 14 der Querseitenwand 7 größer als die Wandstärke der Längsseitenwand 4. Ferner kann zusätzlich oder alternativ die Wandstärke der Querseitenwand 6 größer sein als die Wandstärken der beiden Längsseitenwände 4 und 5.

Aus Figur 5 ergibt sich deutlich, dass die Höhe 16 der konkav gekrümmten Längsseitenwand 4 größer ist als die Höhe 17 der konvex gekrümmten Längsseitenwand 5. Durch diese Maßnahme ist das Zwischenwirbelimplantat 1 keilförmig gestaltet, so dass sich die Ebenen der beiden Anlageflächen 22 und 23 schneiden.

Aus den Figuren 1 und 5 ist außerdem erkennbar, dass im Übergangsbereich 18 der Querseitenwand 6 zur konkav gekrümmten Längsseitenwand 5 eine eine Instrumentenaufnahme 19 bildende Bohrung 20 in der Seitenwand 3, im Speziellen in der Querseitenwand 6 vorgesehen ist. Diese Bohrung 20 ist als Gewindebohrung ausgebildet und besitzt ein Innengewinde 21. An diese Instrumentenaufnahme 19 ist ein Instrument zum Einführen und Einsetzen des Zwischenwirbelimplantats 1 anschließbar.

Da die Gewindebohrung 20 in einem mit einer größeren Wandstärke 14 aufweisenden Bereich der Seitenwand 3, nämlich in der Querseitenwand 6 vorgesehen ist, besteht keine Gefahr der Beschädigung des Innengewindes 21 beim Ansetzen des Instruments bzw. beim Einsetzen des Zwischenwirbelimplantats 1 in den Körper des Patienten.

Ein derartiges Zwischenwirbelimplantat 1 kann problemlos bei einem posterioren Eingriff von dorsal in den Körper eingeführt und von ventral zwischen die beiden Wirbelkörper eingesetzt werden.

## Patentansprüche

1. Zwischenwirbelimplantat zum Einsatz zwischen zwei Wirbelkörper einer Wirbelsäule mit einem zwei Anlageflächen aufweisenden Körper, der als Hohlkörper (2) ausgebildet ist, wobei der Hohlkörper (2) von einer Seitenwand (3) gebildet wird, welche eine konvex gekrümmte Längsseitenwand (4) und eine konkav gekrümmte Längsseitenwand (5) sowie zwei konvex gekrümmte, die beiden Längsseitenwände (4, 5) miteinander verbindende Querseitenwände (6 und 7) aufweist, wobei die Krümmungsradien der Querseitenwände (6 und 7) kleiner sind als die Krümmungsradien der Längsseitenwände (4 und 5), und eine Querseitenwand (6 oder 7) mit einer Instrumentenaufnahme (19) versehen ist, **dadurch gekennzeichnet, dass** die Wandstärke beider Querseitenwände (6 und 7) größer ist als die Wandstärke der Längsseitenwände (4 und 5), und die Instrumentenaufnahme an einem Übergangsbereich (18) von einer Längsseitenwand (5) zu einer Querseitenwand (6) argeordnet ist.

2. Zwischenwirbelimplantat nach Anspruch 1, **dadurch gekennzeichnet, dass** die Seitenwand (3) des Hohlkörpers (2) bohnenförmig gekrümmt ist.

3. Zwischenwirbelimplantat nach Anspruch 1 oder 2, **dadurch gekennzeichnet, dass** die beiden Längsseitenwände (4 und 5) den gleichen Krümmungsradius aufweisen.

4. Zwischenwirbelimplantat nach einem der vorhergehenden Ansprüche, **dadurch gekennzeichnet, dass** die Seitenwand (3) mit Durchbrüchen (10) versehen ist.

5. Zwischenwirbelimplantat nach Anspruch 4, **dadurch gekennzeichnet, dass** die Durchbrüche (10) von Bohrungen (11) gebildet werden.

6. Zwischenwirbelimplantat nach Anspruch 4 oder 5, **dadurch gekennzeichnet, dass** die Seitenwand (3) zwei Reihen (12 und 13) von Durchbrüchen (10) aufweist.

7. Zwischenwirbelimplantat nach Anspruch 6, **dadurch gekennzeichnet, dass** die beiden Reihen (12 und 13) von Durchbrüchen (10) symmetrisch zueinander liegen.

8. Zwischenwirbelimplantat nach einem der vorhergehenden Ansprüche, **dadurch gekennzeichnet, dass** die Querseitenwände (6 und 7) kreisbogenförmig gekrümmt sind.

9. Zwischenwirbelimplantat nach einem der vorhergehenden Ansprüche, **dadurch gekennzeichnet, dass** die Instrumentenaufnahme (19) von einer ein Innengewinde (21) aufweisenden Bohrung (20) gebildet wird.

10. Zwischenwirbelimplantat nach einem der vorhergehenden Ansprüche, **dadurch gekennzeichnet, dass** die Längsseitenwand (5) konkav gekrümmt ist.

11. Zwischenwirbelimplantat nach einem der vorhergehenden Ansprüche, **dadurch gekennzeichnet, dass** die beiden Anlageflächen in winklig zueinander stehenden Ebenen liegen.

12. Zwischenwirbelimplantat nach einem der vorhergehenden Ansprüche, **dadurch gekennzeichnet, dass** die Höhe (17) der konkav gekrümmten Längsseitenwand geringer ist als die Höhe (16) der konvex gekrümmten Seitenwand (4).

13. Zwischenwirbelimplantat nach einem der vorhergehenden Ansprüche, **dadurch gekennzeichnet, dass** die konkav gekrümmte Längsseitenwand (5) dorsal zu liegen kommt.

14. Zwischenwirbelimplantat nach einem der vorhergehenden Ansprüche, **dadurch gekennzeichnet, dass** es aus Metall und/oder Kunststoff und/oder Keramik besteht.

## Claims

1. An intervertebral implant for insertion between two vertebral bodies of a spinal column comprising a body having to two contact surfaces, the body is formed by a hollow body (2), wherein the hollow body (2) is formed by a side wall (3), having a convexly curved longitudinal wall portion (4) , a concavely curved longitudinal wall portion (5), and two convexly curved lateral wall portions (6 and 7) connecting said two longitudinal wall portions (4, 5), whereby the radii of curvature of said lateral wall portions (6 and 7) are smaller than the radii of curvature of said longitudinal wall portions (4 an 5) and one of said two convexly curved lateral wall portions (6 or 7) is provided with an instrument receptacle (19), **characterized in that** the wall thickness of said lateral wall portions (6 and 7) is greater than the wall thickness of said longitudinal wall portions (4 and 5) and said instrument receptacle (19) is situated in an intermediate area (18) between a longitudinal wall portion (5) and a lateral wall portion (6).

2. The intervertebral implant according to claim 1, **characterized in that** said side wall (3) of said hollow body (2) has a kidney-shaped form.

3. The intervertebral implant according to claim 1 or 2, **characterized in that** said longitudinal wall portions (4 and 5) have the same radius of curvature.

4. The intervertebral implant according to one of the preceding claims, **characterized in that** said side wall (3) is provided with openings (10).

5. The intervertebral implant according to claim 4, **characterized in that** said openings (10) are formed by holes (11).

6. The intervertebral implant according to claim 4 or 5, **characterized in that** said side wall (3) is provided with two rows (12 and 13) of openings (10).

7. The intervertebral implant according to claim 6, **characterized in that** said two rows (12 and 13) of openings (10) are arranged symmetrical to each other.

8. The intervertebral implant according to one of the preceding claims, **characterized in that** said lateral wall portions (6 and 7) are curved in the form of a circular arc.

9. The intervertebral implant according to one of the preceding claims, **characterized in that** said instrument receptacle (19) is formed by hole (20) having an internal thread(21).

10. The intervertebral implant according to one of the preceding claims, **characterized in that** longitudinal wall portion (5) is concavely curved.

11. The intervertebral implant according to one of the preceding claims, **characterized in that** said contact surfaces lie in planes that are situated at an angle to each other.

12. The intervertebral implant according to one of the preceding claims, **characterized in that** the height (17) of said concavely curved longitudinal wall portion is smaller than the height (16) of said convexly curved side wall portion (4).

13. The intervertebral implant according to one of the preceding claims, **characterized in that** said concavely curved longitudinal wall portion (5) lies on the dorsal side of the spinal column.

14. The intervertebral implant according to one of the preceding claims, **characterized in that** it consists of metal and/or plastic and/or ceramic.

## Revendications

1. Implant intervertébral destiné à être inséré entre deux corps vertébraux d'une colonne vertébrale, avec un corps à deux faces de contact conçu sous la forme d'un corps creux (2), ledit corps creux (2) étant formé par une paroi (3) qui possède une paroi longitudinale à courbure convexe (4) et une paroi longitudinale à courbure concave (5) ainsi que deux parois transversales à courbure convexe (6 et 7) reliant entre elles les deux parois longitudinales (4, 5), les rayons de courbure des parois transversales (6 et 7) étant inférieurs aux rayons de courbure des parois longitudinales (4 et 5) et une paroi transversale (6 ou 7) présentant un logement (19) destiné à recevoir un instrument, **caractérisé en ce que** l'épaisseur des deux parois transversales (6 et 7) est supérieure à l'épaisseur des parois longitudinales (4 et 5) et **en ce que** le logement destiné à recevoir un instrument est disposé à la jonction (18) entre une paroi longitudinale (5) et une paroi transversale (6).

2. Implant intervertébral selon la revendication 1, **caractérisé en ce que** la paroi (3) du corps creux (2) est courbée en forme de haricot.

3. Implant intervertébral selon la revendication 1 ou la revendication 2, **caractérisé en ce que** les deux parois longitudinales (4 et 5) possèdent le même rayon de courbure.

4. Implant intervertébral selon l'une des revendications précédentes, **caractérisé en ce que** la paroi (3) présente des ouvertures (10).

5. Implant intervertébral selon la revendication 4, **caractérisé en ce que** les ouvertures (10) consistent en des perçages (11).

6. Implant intervertébral selon la revendication 4 ou la revendication 5, **caractérisé en ce que** la paroi (3) comporte deux rangées (12 et 13) d'ouvertures (10).

7. Implant intervertébral selon la revendication 6, **caractérisé en ce que** les deux rangées (12 et 13) d'ouvertures (10) sont disposées symétriquement.

8. Implant intervertébral selon l'une des revendications précédentes, **caractérisé en ce que** les parois transversales (6 et 7) sont courbées en arc de cercle.

9. Implant intervertébral selon l'une des revendications précédentes, **caractérisé en ce que** le logement (19) destiné à recevoir un instrument consiste en un perçage (20) doté d'un taraudage (21).

10. Implant intervertébral selon l'une des revendications précédentes, **caractérisé en ce que** la paroi longitudinale (5) possède une courbure concave.

11. Implant intervertébral selon l'une des revendications précédentes, **caractérisé en ce que** les deux faces de contact se trouvent dans deux plans formant un angle entre eux.

12. Implant intervertébral selon l'une des revendications précédentes, **caractérisé en ce que** la hauteur (17) de la paroi longitudinale à courbure concave est inférieure à la hauteur (16) de la paroi à courbure convexe (4).

13. Implant intervertébral selon l'une des revendications précédentes, **caractérisé en ce que** la paroi longitudinale à courbure concave (5) est située dorsalement.

14. Implant intervertébral selon l'une des revendications précédentes, **caractérisé en ce qu'**il est en métal et/ou en plastique et/ou en céramique.
